# EUROPEAN PATENT SPECIFICATION

(11) **EP 4 539 942 B1**
(45) Date of publication and mention of the grant of the patent: **25.03.2026**
(21) Application number: 23734752.1
(22) Date of filing: 13.06.2023
(51) Int. Cl.: A61Q 11/02, A61K 8/20, A61K 8/22, A61K 8/40, A61K 8/67, A61K 8/9711

(54) **TOOTH WHITENING KIT**
ZAHNAUFHELLUNGSSET
KIT DE BLANCHIMENT DENTAIRE

(30) Priority: 15.06.2022 IT 202200012629
(43) Date of publication of application: 23.04.2025
(73) Proprietor: IDS RESEARCH SOCIETÀ A RESPONSABILITÀ LIMITATA, 16122 Genova (IT)
(72) Inventor: PIERGALLINI, Remigio, 63066 GROTTAMMARE (AP) (IT); LOUPIS, Nikolaos, 14562 KIFISIA (GR)
(74) Representative: Cutropia, Gianluigi
(86) International application number: PCT/IB2023/056089
(87) International publication number: WO 2023/242732

(56) References cited:
- WO-A1-2011/084746
- WO-A1-2015/196272
- WO-A1-2018/224311
- WO-A1-2020/018089
- US-A1- 2009 238 778

## Description

The present invention relates to a dental whitening kit comprising a hydrogen peroxide gel and a photo-activator gel.

Peroxides are generally used in the form of gels for the whitening of human teeth. They can be applied at home with the use of custom-made night masks or in the dental surgery where they are applied directly onto the tooth surface by the dental surgery staff.

Peroxides of different compositions gradually decompose, releasing molecular oxygen and active forms of oxygen. The active forms normally undergo a spontaneous degradation, but they are not abundant due to the action of antioxidant enzymes in the saliva and the gingival crevicular fluid.

The ingredients of a whitening gel used in the dental surgery must be more aggressive in order to emit more active forms of oxygen in less time and immediately achieve an acceptable whitening result in one dental session of average duration. In order to accomplish such a purpose, the ingredients must be generally activated with the use of physical or chemical activators or catalysts.

The most commonly used chemical activators are various metal ions, such as iron ions or the like, such as alkaline solutions mixed with peroxide gel. Peroxides decompose more rapidly in an alkaline environment, generating oxygen, even though the presence of radical forms of oxygen is limited.

With reference to the prior art, it has been noted that the active forms of oxygen are much more effective for dental whitening. The more radicals are produced, the better the whitening effect will be.

Photoabsorbent molecules, such as biologically acceptable dyes (CRAS: commonly recognized as safe), are used to intensify the dental whitening effect and to amplify active oxygen production.

Certain colors selectively absorb different wavelengths of emitted light (from LEDs or lasers) and so they accumulate energy that is consecutively released into the environment and also into the peroxides contained in the compositions, catalyzing the production of active forms of oxygen. It has been proven that luminous energy also intensifies the production of free radicals. In such a case, the whitening gel should be used together with light-emitting devices, such as LED lamps or lasers, that emit blue light in the 480 to 500 nm range and are commonly found in a dental surgery.

WO2020/018089A1 discloses an oral care composition according to the preamble of independent claim 1.

The purpose of the present invention is to eliminate the drawbacks of the prior art by providing a dental whitening kit that is capable of activating the oxygen contained in a hydrogen peroxide gel, creating reactive forms of oxygen.

Another purpose is to provide such a dental whitening kit that is practical, reliable, efficient and effective.

These purposes are achieved in accordance with the invention with the features of the appended independent claim 1

Advantageous achievements of the invention appear from the dependent claims.

Further features of the invention will appear clearer from the following detailed description, which refers to a merely illustrative and therefore nonlimiting embodiment, illustrated in the following examples.

The dental whitening kit according to the invention comprises a hydrogen peroxide gel and a photo-activator gel.

The hydrogen peroxide gel contains an amount of hydrogen peroxide (H₂O₂) in a weight percentage comprised between 5% and 50% relative to the total weight of the gel.

The hydrogen peroxide gel comprises a hydrogen peroxide aqueous solution and a gelling composition.

By way of example, the gelling composition comprises:
- colloidal silicon dioxide in amorphous form,
- potassium and aluminum silicate, and
- crystalline aluminum dioxide.

The hydrogen peroxide aqueous solution comprises hydrogen peroxide (H₂O₂) in a weight percentage comprised between 10% and 50% relative to the total weight of the aqueous solution.

Colloidal silicon dioxide is also known as hydrophilic pyrogenic silica because it is obtained by hydrolysis in gaseous phase of silicon tetrachloride (SiCl4). The colloidal silicon dioxide absorbs and retains water. The colloidal silicon dioxide is in the amorphous form (random distribution of atoms). This means that, unlike crystalline silica, it is not dangerous in case of accidental inhalation. The colloidal silicon dioxide is in the form of micronized powder with a particle size of 0.2-0.3 µm.

The hydrogen peroxide gel comprises colloidal silicon dioxide in a weight percentage comprised between 0.1% and 7%, preferably 2%-5%, relative to the total weight of the gel.

The potassium and aluminum silicate preferably comprises mica powder in crystalline form. Mica crystals are generally tabular with diamond or hexagonal section with angles of approximately 60° and 120°, and assume pseudo-orthorhombic or pseudo-hexagonal morphologies
The potassium and aluminum silicate can also be a food additive, known as E555, used as a carrier of E171 (titanium dioxide) and E172 (iron oxides and hydroxides) dyes.

The hydrogen peroxide gel comprises potassium and aluminum silicate in a weight percentage comprised between 0.1% and 5%, preferably 1%-3%, relative to the total weight of the gel.

Aluminum dioxide (Al203) in crystalline form is preferably corundum powder. Corundum has a crystalline form with tabular, or prismatic or barrel habitus (by bipyramid sequence).

The hydrogen peroxide gel comprises aluminum dioxide in a weight percentage comprised between 0.1% and 5%, preferably 1%-3%, relative to the total weight of the gel.

In order to obtain the whitening gel, firstly the solid components (colloidal silicon dioxide, potassium and aluminum silicate, and aluminum dioxide) are mixed.

Then, the mixture of solid components is poured into the hydrogen peroxide aqueous solution, and a planetary mixing with a mixer is performed. In this way a product in the form of a gel is obtained.

With the addition of the hydrogen peroxide aqueous solution, the crystalline molecules contained in the mixture of solid components produce a gel, in which the hydrogen peroxide aqueous solution is attracted by the crystalline molecules so as to surround said crystalline molecules, forming an atmosphere that surrounds the crystalline molecules. Light can penetrate such a hydrogen peroxide atmosphere, increasing the decomposition of the hydrogen peroxide into water and oxygen, via the transition of the oxygen to reactive oxygen species (ROS).

The photo-activator gel comprises:
- purified water,
- glycerin,
- a gelling agent, and
- photo-activator substances that act as catalysts when they are exposed to blue light, in a decomposition reaction of the hydrogen peroxide into water and oxygen, increasing the generation of reactive oxygen species (ROS).

The photo-activator substances comprise:
- red azo dye,
- vitamin B2,
- Fucus vesiculosus seaweed, and
- potassium iodide.

The red azo dye preferably comprises Allura Red, which is an azo dye, is water-soluble with strongly polar characteristics, which selectively absorbs blue light from 480 to 500 nm, i.e. the wavelengths used in dental whitening lamps, strongly increasing the proton energy in the decomposition reaction of the hydrogen peroxide (H2O2) into water (H2O) and oxygen (O2). The photo-activator gel contains azo dyes in a weight percentage comprised between 0.001% and 1% of the total weight of the photo-activator gel.

Vitamin B2 is preferably riboflavin. Vitamin B2 is a water-soluble vitamin and an endogenous photo-sensitizer. When exposed to the blue light emitted by the dental lamps, vitamin B2 acts as a photo-catalyst for the hydrogen peroxide so as to favor a generation of reactive oxygen species (ROS) during the decomposition of the hydrogen peroxide into oxygen and water. The photo-activator gel contains vitamin B2 in a weight percentage comprised between 0.01% to 2% relative to the total weight of the photo-activator gel.

Fucus vesiculosus seaweed (INCI name) comprises a natural algae extract that strongly absorbs the blue light from the dental lamps, leading to the formation of oxygen free radicals from the decomposition of the hydrogen peroxide. The photo-activator gel contains Fucus vesiculosus seaweed in a weight percentage comprised between 0.1% and 5% of the total weight of the photo-activator gel.

Potassium iodide (KI) enhances the photo-catalyzing action of the Fucus vesiculosus seaweed and increases the production of reactive oxygen species (ROS) during the decomposition of the hydrogen peroxide into oxygen and water. The photo-activator gel contains potassium iodide in a weight percentage comprised between 0.0001% and 0.001% relative to the total weight of the photo-activator gel.

The above-mentioned four photo-activator substances contribute to a synergistic action to maximize the production of reactive oxygen species (ROS) during the decomposition of the hydrogen peroxide into oxygen and water when the final gel obtained from the kit according to the invention is activated with a blue light emitted from a dental lamp.

Advantageously, the photo-activator gel also comprises citronellol to further increase the production of reactive oxygen species (ROS). Citronellol (C₁₀H₂₀O) is a natural acyclic monoterpenoid that contains molecules with selective oxidation in the presence of hydrogen peroxide. Thus, citronellol oxidizes when it comes in contact with the hydrogen peroxide contained in the hydrogen peroxide gel, generating reactive forms of oxygen that contribute to dental whitening. The photo-activator gel contains citronellol in a weight percentage comprised between 0.001% and 0.01% relative to the total weight of the photo-activator gel.

In view of the fact that the photo-activator gel contains organic substances, such as Fucus vesiculosus seaweed, advantageously such a photo-activator gel may comprise an antimicrobial agent, such as phenoxyethanol, in a weight percentage comprised between 0.1% and 1% relative to the total weight of the photo-activator gel.

Considering that the photo-activator gel contains a mineral, such as potassium iodide, advantageously such a photo-activator gel may comprise a chelating agent, such as ethylenediaminetetraacetic acid (EDTA), in a weight percentage comprised between 0.01% and 2% relative to the total weight of the photo-activator gel.

The mixing of purified water, glycerin and gelling agent forms a gel with the desired consistency. The gelling agent may comprise a vinyl polymer (Carbopol) in a weight percentage comprised between 0.1% and 3% relative to the total weight of the gel.

The amount of water and glycerin can vary widely depending on the desired gel consistency. By way of example, the photo-activator gel comprises glycerin in a weight percentage comprised in the range from 10% and 60% of the total weight of the photo-activator gel.

To obtain the photo-activator gel, the liquid substances (water, glycerin, and possibly phenoxyethanol) are mixed together to make a liquid mixture.

The solid substances (photo-activator substances and gelling agent) are mixed together to make a solid mixture. Then the solid mixture is mixed with the liquid mixture to obtain the photo-activator gel.

The hydrogen peroxide gel and the photo-activator gel are kept in two separate packages and are mixed before use. The hydrogen peroxide gel and the photo-activator gel are mixed together in a ratio in the range of 1:20 to 1:1 of hydrogen peroxide gel and photo-activator gel. The mixing of the two gels can be done in various ways.

Two syringes connected to each other by means of a connector can be used. A first syringe contains the hydrogen peroxide gel and a second syringe contains the photo-activator gel. The two syringes are connected to each other via the connector, and the two gels are mixed with back and forth movements of the plungers of the syringes until the final gel has a uniform consistency and color. The final gel is held in one of the syringes and is then applied directly onto the teeth to be whitened.

Another method for mixing and preparing the final gel may be with a bi-chamber syringe, that is, two syringes connected together in one system. The plungers of the syringes are actuated simultaneously with a pressure apparatus. The contents of the syringes is mixed at the outlet of the bi-chamber syringe through a mixing nozzle containing a mixing spiral. The final gel obtained by mixing the hydrogen peroxide gel and the photo-activator gel is applied directly onto the teeth to be whitened.

After applying the final gel, a blue light is directed onto the final gel to activate the photo-activator substances that react with the hydrogen peroxide, acting as catalysts for the decomposition of the hydrogen peroxide into water and oxygen and for the generation of reactive oxygen species (ROS) that contribute to tooth whitening.

The blue light is emitted by dental lamps of known type.

## Claims

1. Dental whitening kit comprising a hydrogen peroxide gel and a photo-activator gel suitable for being mixed and applied to teeth to be whitened;
wherein said photo-activator gel comprises:
- purified water,
- glycerin, and
- a gelling agent,
**characterized in that**
said photo-activator gel comprises photo-activator substances that act as catalysts when they are exposed to blue light, in a decomposition reaction of the hydrogen peroxide into water and oxygen, increasing the generation of reactive oxygen species (ROS);
wherein said photo-activator substances comprise:
- red azoic dye,
- vitamin B2,
- Fucus vesiculosus seaweed, and
- potassium iodide.

2. The kit according to claim 1, wherein said red azoic dye comprises Allura Red.

3. The kit according to claim 1 or 2, wherein said photo-activator gel contains azoic dye in a weight percentage comprised between 0.001% and 1% relative to the total weight of the photo-activator gel.

4. The kit according to any one of the preceding claims, wherein said vitamin B2 is riboflavin.

5. The kit according to any one of the preceding claims, wherein said photo-activator gel contains vitamin B2 in a weight percentage comprised between 0.01% and 2% relative to the total weight of the photo-activator gel.

6. The kit according to any one of the preceding claims, wherein said photo-activator gel comprises Fucus vesiculosus seaweed in a weight percentage comprised between 0.1% and 5% relative to the total weight of the photo-activator gel.

7. The kit according to any one of the preceding claims, wherein said photo-activator gel comprises potassium iodide in a weight percentage comprised between 0.0001% and 0.001% relative to the total weight of the photo-activator gel.

8. The kit according to any one of the preceding claims, wherein said photo-activator gel comprises citronellol to further increase the production of reactive oxygen species (ROS).

9. The kit according to claim 8, wherein said photo-activator gel comprises citronellol in a weight percentage comprised between 0.001% and 0.01% relative to the total weight of the photo-activator gel.

10. The kit according to any one of the preceding claims, wherein said photo-activator gel comprises an antimicrobial agent, such as phenoxyethanol, in a weight percentage comprised between 0.1% and 1% relative to the total weight of the photo-activator gel.

11. The kit according to any one of the preceding claims, wherein said photo-activator gel comprises a chelating agent, such as ethylenediaminetetraacetic acid (EDTA), in a weight percentage comprised between 0.01% and 2% relative to the total weight of the photo-activator gel.

## Patentansprüche

1. Kit zur Zahnaufhellung, umfassend ein Gel auf Wasserstoffperoxidbasis und ein Lichtaktivierungsgel, die dazu bestimmt sind, miteinander vermischt und auf die zu bleichenden Zähne aufgetragen zu werden;
wobei das Lichtaktivierungsgel umfasst:
- gereinigtes Wasser,
- Glyzerin,
- ein Geliermittel und
- Lichtaktivierungsstoffe, welche, wenn sie blauem Licht ausgesetzt werden, als Katalysatoren in einer Reaktion wirken, in der das Wasserstoffperoxid in Wasser und Sauerstoff zerfällt und die Entstehung reaktiver Sauerstoffspezies (ROS) erhöht wird;
wobei die Lichtaktivierungsstoffe umfassen:
- roter Azofarbstoff,
- Vitamin B2,
- Blasentang (Fucus vesiculosus) und
- Kaliumiodid.

2. Kit nach Anspruch 1, wobei der rote Azofarbstoff Allurarot umfasst.

3. Kit nach Anspruch 1 oder 2, wobei das Lichtaktivierungsgel Azofarbstoff in einem Gewichtsprozentsatz im Bereich von 0,001 bis 1 %, bezogen auf das Gesamtgewicht des Lichtaktivierungsgels, enthält.

4. Kit nach einem der vorstehenden Ansprüche, wobei das Vitamin B2 Riboflavin ist.

5. Kit nach einem der vorstehenden Ansprüche, wobei das Lichtaktivierungsgel Vitamin B2 in einem Gewichtsprozentsatz im Bereich von 0,01 bis 2 %, bezogen auf das Gesamtgewicht des Lichtaktivierungsgels, enthält.

6. Kit nach einem der vorstehenden Ansprüche, wobei das Lichtaktivierungsgel Blasentang (Fucus vesiculosus) in einem Gewichtsprozentsatz im Bereich von 0,1 bis 5 %, bezogen auf das Gesamtgewicht des Lichtaktivierungsgels, umfasst.

7. Kit nach einem der vorstehenden Ansprüche, wobei das Lichtaktivierungsgel Kaliumiodid in einem Gewichtsprozentsatz im Bereich 0,0001 bis 0,001 %, bezogen auf das Gesamtgewicht des Lichtaktivierungsgels, umfasst.

8. Kit nach einem der vorstehenden Ansprüche, wobei das Lichtaktivierungsgel Citronellol umfasst, um die Entstehung von reaktiven Sauerstoffspezies (ROS) weiter zu erhöhen.

9. Kit nach Anspruch 8, wobei das Lichtaktivierungsgel Citronellol in einem Gewichtsprozentsatz im Bereich von 0,001 bis 0,01 %, bezogen auf das Gesamtgewicht des Lichtaktivierungsgels, umfasst.

10. Kit nach einem der vorstehenden Ansprüche, wobei das Lichtaktivierungsgel einen antimikrobiellen Wirkstoff wie Phenoxyethanol in einem Gewichtsprozentsatz im Bereich von 0,1 bis 1 %, bezogen auf das Gesamtgewicht des Lichtaktivierungsgels, umfasst.

11. Kit nach einem der vorstehenden Ansprüche, wobei das Lichtaktivierungsgel einen Chelatbildner wie Ethylendiamintetraessigsäure (EDTA) in einem Gewichtsprozentsatz im Bereich 0,01 bis 2 %, bezogen auf das Gesamtgewicht des Lichtaktivierungsgels, umfasst.

## Revendications

1. Kit de blanchiment dentaire comprenant un gel à base de peroxyde d'hydrogène et un gel photo-activateur destinés à être mélangés entre eux et appliqués sur les dents à blanchir ;
où ledit gel photo-activateur comprend :
- de l'eau purifiée,
- de la glycérine, et
- un agent gélifiant,
**caractérisé en ce que**
ledit gel photo-activateur comprend des substances photo-activatrices qui, lorsqu'elles sont exposées à la lumière bleue, agissent en tant que catalyseurs dans une réaction de décomposition du peroxyde d'hydrogène en eau et oxygène, en augmentant la génération de formes réactives d'oxygène (ROS) ;
où lesdites substances photo-activatrices comprennent :
- un colorant azoïque rouge,
- de la vitamine B2,
- une algue marine Fucus Vescicolosus, et
- de l'iodure de potassium.

2. Kit selon la revendication 1, où ledit colorant azoïque rouge comprend du Rouge allura.

3. Kit selon la revendication 1 ou 2, où ledit gel photo-activateur contient du colorant azoïque dans un pourcentage poids compris entre 0,001% - 1% par rapport au poids total du gel photo-activateur.

4. Kit selon l'une quelconque des revendications précédentes, où ladite vitamine B2 est de la riboflavine.

5. Kit selon l'une quelconque des revendications précédentes, où ledit gel photo-activateur contient de la vitamine B2 dans un pourcentage poids compris entre 0,01% - 2% par rapport au poids total du gel photo-activateur.

6. Kit selon l'une quelconque des revendications précédentes, où ledit gel photo-activateur comprend de l'algue marine du type fucus vescicolosus dans un pourcentage poids compris entre 0,01% - 5% par rapport au poids total du gel photo-activateur.

7. Kit selon l'une quelconque des revendications précédentes, où ledit gel photo-activateur comprend de l'iodure de potassium dans un pourcentage poids compris entre 0,0001% - 0,001% par rapport au poids total du gel photo-activateur.

8. Kit selon l'une quelconque des revendications précédentes, où ledit gel photo-activateur comprend du citronellol pour augmenter ultérieurement la production d'oxygène réactif (ROS).

9. Kit selon la revendication 8, où ledit gel photo-activateur comprend du citronellol dans un pourcentage poids compris entre 0,001% - 0,01% par rapport au poids total du gel photo-activateur.

10. Kit selon l'une quelconque des revendications précédentes, où ledit gel photo-activateur comprend un agent antimicrobien, tel que le phénoxyéthanol, dans un pourcentage poids compris entre 0,1% - 1% par rapport au poids total du gel photo-activateur.

11. Kit selon l'une quelconque des revendications précédentes, où ledit gel photo-activateur comprend un agent de chélation, tel que l'acide éthylènediamine-tétracétique (EDTA), dans un pourcentage poids compris entre 0,01% - 2% par rapport au poids total du gel photo-activateur.
